# EUROPEAN PATENT APPLICATION

(11) **EP 1 142 908 A1**
(43) Date of publication of application: **10.10.2001**
(21) Application number: 00900127.2
(22) Date of filing: 06.01.2000
(51) Int. Cl.: C07K 14/47, C12P 21/02, C12N 15/12

(54) **PROCESS FOR PRODUCING ACTIVATED PROTEIN**

(30) Priority: 07.01.1999 JP 184099; 11.01.1999 JP 442699
(71) Applicant: Takeda Chemical Industries, Ltd., Osaka-shi, Osaka 541-0045 (JP)
(72) Inventor: ITO, Takashi, Kobe-shi Hyogo 658-0032 (JP); TANAKA, Yoko, Kyotanabe-shi Kyoto 610-0331 (JP); KAISHO, Tomoko, Osaka-shi Osaka 532-0013 (JP); NISHIMURA, Osamu, Tsukuba-shi Ibaraki 305-0812 (JP)
(74) Representative: Best, Michael, Dr.
(86) International application number: JP0000024
(87) International publication number: WO0040610

(57) **Abstract**

The present invention relates to a process for activating a purified polypeptide, characterized by bringing the purified polypeptide having an amino acid sequence identical or substantially identical to the amino acid sequence represented by SEQ ID NO: 1 into contact with copper ions, calcium ions or magnesium ions, or a substance providing copper ions, a substance providing calcium ions, or a substance providing magnesium ions.

The methods for activation or manufacturing of the present invention enable the inexpensive mass production of active MPIF-1Δ23 which protects myeloid stem cells from chemotherapeutic drugs and permits rapid recovery from leukopenia, which is a limiting factor in the administration of anti-tumor agents.

## Description

### TECHNICAL FIELD

The present invention relates to process for activating a polypeptide through genetic recombinant techniques using *E. coli*, a method for manufacturing an activated polypeptide of chemokine through genetic recombinant techniques using *E. coli*, and the like.

### BACKGROUND ART

*E. coli* is most widely used for economic reasons and the like to produce recombinant proteins (polypeptides), but expression with *E. coli* can result in the formation of inclusions and can fail to produce the proper S-S bonds, so the expected activity is sometimes not obtained, even when proteins are obtained. In one method for obtaining active proteins using *E*. *coli*, a secretory signal allows proteins in soluble form to accumulate in the periplasmic space, thus giving active proteins (C.N. Change et al, *Gene,* **55**, 189-196, (1987)).

On the other hand, an advantage of expression in the form of inclusions with *E. coli* is that the target protein can be obtained in large amounts and with high purity in inclusions. However, obtaining the active forms from inclusions requires a refolding process comprising the solubilization of the inclusions with a denaturant and the removal of the denaturant (R.H. Pain, ed., *Folding of Proteins,* 245-279 (1995), Schpringer-Verlag Tokyo). The use of a precipitation preventive such as urea or arginine hydrochloride and the use of a redox buffer in such refolding have been reported to improve refolding efficiency. However, the conditions differ drastically depending on the target protein (polypeptide), and in many cases active forms are not obtained by general refolding methods.

There is a need to establish a simpler and more reliable way of obtaining active forms in the production of recombinant proteins (polypeptides). MPIF-1Δ23 (WO 95/17092) has action in suppressing colony formation of myeloid stem cells, thus protecting them from chemotherapeutic drugs, and is expected to prove effective in permitting rapid recovery from leukopenia, which is a limiting factor in the administration of anti-tumor agents. There is a need to establish a method for the inexpensive mass production of active MPIF-1Δ23 for when MPIF-1Δ23 is used in the form of such a drug.

### DISCLOSURE OF THE INVENTION

As a result of extensive research on efficient ways of obtaining the active form of MPIF (myeloid progenitor inhibitory factor)-1Δ23, a type of CC chemokine, the inventors found that such an objective can be achieved by treating MFIF-1Δ23 with copper, calcium, or magnesium.

That is, the present invention relates to:
(1) A process for activating a purified polypeptide, characterized by bringing a purified polypeptide having an amino acid sequence that is identical or substantially identical to the amino acid sequence represented by SEQ ID NO: 1 into contact with copper ions, calcium ions, or magnesium ions, or a substance providing copper ions, a substance providing calcium ions, or a substance providing magnesium ions;
(2) A process according to (1) above, wherein the substance providing copper ions is copper, copper sulfate, copper bromide, copper fluoride, copper oxalate, copper chloride, copper formate, copper acetate, or copper sulfide;
(3) A process according to (2) above, wherein the substance providing copper ions is copper sulfate;
(4) A process according to (1) above, wherein the substance providing calcium ions is calcium, calcium sulfate, calcium acetate, calcium nitrate, calcium formate, or calcium chloride;
(5) A process according to (4) above, wherein the substance providing calcium ions is calcium chloride;
(6) A process according to (1) above, wherein the substance providing magnesium ions is magnesium, magnesium sulfate, magnesium acetate, magnesium nitrate, magnesium formate, or magnesium chloride;
(7) A process according to (6) above, wherein the substance providing the magnesium ion is magnesium sulfate;
(8) A process according to (1) above, wherein the activity of the activated polypeptide is activity in suppressing cell differentiation;
(9) A process for producing an active polypeptide, characterized by bringing a purified polypeptide having an amino acid sequence that is identical or substantially identical to the amino acid sequence represented by SEQ ID NO: 1 into contact with copper ions, calcium ions, or magnesium ions, or a substance providing copper ions, a substance providing calcium ions, or a substance providing magnesium ions; and
(10) A polypeptide, or an amide thereof, or an ester thereof, or a salt thereof, which has been activated by means of the activating process according to (1) above.

### BRIEF DESCRIPTION OF THE DRAWINGS

Figure 1 is an electrophorogram giving the results of SDS-PAGE of the MPIF-1△23 prepared in Reference Example 2;
Figure 2 shows the levels of activation of MPIF-1Δ23 treated with copper sulfate in Example 1;
Figure 3 shows the results obtained in the assay of the action of Des-Met MPIF-1Δ23 in suppressing mouse myeloid cell differentiation in Example 2;
Figure 4 shows the changes in the intracellular calcium concentration of Des-Met MPIF-1Δ23 in Example 2; and
Figure 5 shows the results obtained in the assay of the action of MPIF-1Δ23 in suppressing mouse myeloid cell differentiation in Example 2.

### BEST MODE FOR CARRYING OUT THE INVENTION

Examples of polypeptides used in the activation method of the present invention or the method for manufacturing active polypeptides (hereinafter sometimes referred to as the polypeptides of the present invention) include polypeptides with an amino acid sequence that is identical or substantially identical to the amino acid sequence represented by SEQ ID NO: 1.

The polypeptides of the present invention may be polypeptides derived from recombinant *E. coli* or synthetic polypeptides.

Examples of amino acid sequences that are substantially identical to the amino acid sequence represented by SEQ ID NO: 1 include amino acid sequences with at least about 80%, preferably at least about 90%, and even more preferably at least about 95% homology with the amino acid sequence represented by SEQ ID NO: 1.

Examples of polypeptides with an amino acid sequence that is substantially identical to the amino acid sequence represented by SEQ ID NO: 1 preferably include polypeptides which have an amino acid sequence substantially identical to the amino acid sequence represented by SEQ ID NO: 1 and which, as a result of the activation or production method of the present invention, have activity substantially identical to that of polypeptides represented by the amino acid sequence of SEQ ID NO: 1.

Examples of substantially identical activity include activity in suppressing cell differentiation (such as suppression of myeloid cell differentiation).

Substantially identical activity means activity that is substantially identical. Accordingly, cell differentiation-suppressing activity or the like should be the same (such as about 0.01 to 100-fold, preferably about 0.5 to 20-fold, and more preferably about 0.5 to 2-fold), although the quantitative elements involved in the level of such activity or the molecular weight of the polypeptides may vary.

The activity in suppressing cell differentiation (such as suppression of myeloid cell differentiation) can be assayed according to publicly known methods (such as *J*. *Exp. Med.,* **185**, 1163-1172 (1997)).

Activated polypeptides of the present invention indicate polypeptides of the present invention in which, in terms of the action in suppressing myeloid cell differentiation (particularly the mouse myeloid cell differentiation-suppressing activity described in the examples below), the dose showing 30% LPP-CFC colony forming suppression is 1/5 or less, and preferably 1/20 or less, compared to that of polypeptides of the present invention before activation.

Examples of polypeptides of the present invention include polypeptides having: (1) amino acid sequences with one or more (preferably 1 to about 30, more preferably 1 to about 10, and even more preferably several (1 or 2)) amino acid deletions in the amino acid sequence represented by SEQ ID NO: 1; (2) amino acid sequences with one or more (preferably 1 to about 30, more preferably 1 to about 10, and even more preferably several (1 or 2)) amino acid additions in the amino acid sequence represented by SEQ ID NO: 1; (3) amino acid sequences with one or more (preferably 1 to about 30, more preferably 1 to about 10, and even more preferably several (1 or 2)) amino acid substitutions with other amino acids in the amino acid sequence represented by SEQ ID NO: 1; and (4) amino acid sequences in the combination with the above.

In accordance with the usual procedure for designating peptides, the left terminal of the polypeptides in the present Specification is referred to as the N terminal (amino terminal), and the right terminal is referred to as the C terminal (carboxyl terminal). The C terminal of polypeptides of the present invention, including receptor proteins having the amino acid sequence represented by SEQ ID NO: 1, is usually a carboxyl group (-COOH) or carboxylate (-COO⁻), but the C terminal may also be an amide (-CONH₂) or ester (-COOR).

Examples of R in such esters include C₁₋₆ alkyl groups such as methyl, ethyl, n-propyl, isopropyl, or n-butyl, C₃₋₈ cycloalkyl groups such as cyclopentyl and cyclohexyl, C₆₋₁₂ aryl groups such as phenyl and α-naphthyl, phenyl-C₁₋₂ alkyls such as benzyl and phenethyl, or α-naphthyl-C₁₋₂ alkyls such as benzyl or phenethyl, α-naphthyl-C₁₋₂ alkyls such as α-naphthylmethyl and other such C₇₋₁₄ aralkyl groups, and pivaloyloxymethyl groups commonly used as oral esters.

The polypeptides of the present invention also include polypeptides, which have carboxyl or carboxylate groups in addition to those in the C terminal, where such groups are amidated or esterified. Examples of esters in such cases include the aforementioned esters of the aforementioned C terminal.

The polypeptides of the present invention also include conjugated proteins such as those in which the amino group of the methionine residue of the N terminal among the aforementioned polypeptides is protected by a protective group (formyl groups, acetyl groups or other C₂₋₆ alkanoyl groups and other such C₁₋₆ acyl groups); those in which the N terminal side is endogenously cleaved, and the glutamyl group that is produced is pyroglutamated; those in which substituents on the side chains of amino acids in the molecule (such as -OH, -SH, amino groups, imidazole group, indole groups, and guanidino groups) are protected by suitable protective groups (such as formyl groups, acetyl groups or other C₂₋₆ alkanoyl groups and other such C₁₋₆ acyl groups); or what are referred to as glycoproteins with sugar chains bonded thereto.

Specific examples of the polypeptides in the present invention include those with the amino acid sequence represented by SEQ ID NO: 1.

The polypeptides of the present invention may form salts. Examples of salts of the polypeptides of the present invention preferably include pharmaceutically acceptable salts with acids. Examples of such salts include salts with inorganic acids (such as hydrochloric acid, phosphoric acid, hydrobromic acid, and sulfuric acid) or with organic acids (such as acetic acid, formic acid, propionic acid, fumaric acid, maleic acid, succinic acid, tartaric acid, citric acid, malic acid, oxalic acid, benzoic acid, methanesulfonic acid, benzenesulfonic acid).

The process for activating the polypeptides of the present invention and the method for manufacturing activated polypeptides of the present invention are described in detail below.

Means for cloning DNA fully encoding the polypeptides of the present invention include amplification by PCR using synthetic primer with a partial base sequence for the polypeptide of the present invention, or screening by means of hybridization of DNA incorporated into a suitable vector with labeled DNA fragments or synthetic DNA encoding part or all of the polypeptide of the present invention. The hybridization may be undertaken in accordance with techniques such as that in *Molecular Cloning* (2^{nd} ed. J. Sambrook et al., Cold Spring Harbor Lab. Press (1989)). Commercially available DNA libraries can be used in accordance with the accompanying protocol.

DNA base sequences can be substituted using a publicly known kit such as Mutan™-G (by Takara Shuzo) or Mutan™-K (Takara Shuzo) in accordance with publicly known methods, such as the gapped duplex method or the Kunkel method, or the modification thereof.

Cloned polypeptide-encoding DNA may be used as such or after digestion with suitable restriction enzymes or after the addition of linker DNA, depending on the purpose. The DNA may have ATG as the translation start codon on the 5' terminal side, and TAA, TGA, or TAG as the translation stop codon on the 3' terminal side. The translation start and stop codons can be added using suitable synthetic DNA adapters.

Expression vectors for the polypeptides of the present invention can be manufactured by (1) exciting target DNA fragments from the DNA encoding a polypeptide of the present invention, and (2) ligating the DNA fragments downstream of a promoter in a suitable expression vector.

Vectors which may be used include pBR322, pBR325, pUC12, and pUC13.

Examples of preferred promoters include the trp promoter, lac promoter, recA promoter, λPL promoter, and lpp promoter.

In addition to the above, expression vectors can also include enhancers, splicing signals, polyA signals, and selection markers. Examples of selection markers include the ampicillin resistance gene (hereinafter sometimes referred to as Amp^{r}), and the neomycin resistance gene (G418 resistance, hereinafter sometimes referred to as Neo).

Transformants can be manufactured using such vectors containing DNA encoding the polypeptides of the present invention.

Cells such as *E. coli* can be used, for example, as the host.

Specific examples of *E. coli* (Escherichia coli) include *E. coli* K12-DH1 (*Proc. Natl. Acad*. *Sci*. *USA*), **60**:160 (1968)), JM103 (*Nucleic Acids Research,* **9:**309 (1981)), JA221 (*Journal of Molecular Biology,* **120**:517 (1978)), HB101 (*Journal of Molecular Biology,* **41:**459 (1969)), C600 (*Genetics,* **39**:440 (1954)), and MM294 and MM294 (DE3).

*E. coli* can be transformed, for example, by a method such as that in *Proc. Natl. Acad. Sci. USA,* **69:**2110 (1972) or *Gene*, **17:**107 (1982).

When culturing transformants with *E. coli* hosts, liquid media are preferred for the culture, and should be prepared in such a way as to contain carbon sources, nitrogen sources, inorganic material, and other materials necessary for the growth of the transformants. Carbon sources include glucose, dextrins, soluble starches, and sucrose, and nitrogen sources include inorganic or organic substances such as ammonium salts, nitrates, corn steep liquor, peptone, casein, meat extract, soybean cake, and potato extract. Examples of inorganic materials include calcium chloride, sodium dihydrogen phosphate, and magnesium chloride. Yeast extracts, vitamins, growth promoting factors, and the like may also be added. The medium pH is preferably about 5 to 8.

A preferred media for culturing *E. coli* is M9 medium containing glucose and casamino acid (Miller, *Journal of Experiments in Molecular Genetics,* pp. 431-433, Cold Spring Harbor Laboratory, New York (1972)). A chemical such as 3β-indoleacrylic acid can be added to enhance the promoter as needed.

In cases where the host is *E. coli*, the culture usually takes about 3 to 24 hours at about 15 to 43°C. The culture can be aerated or stirred as needed.

Polypeptides of the present invention can be isolated and purified from the aforementioned cultures in the following manner, for example.

When polypeptides of the present invention are extracted from cultured bacterial cells, the bacterial cells are collected by a publicly known method following the culture and are suspended in a suitable buffer, they are disrupted by ultrasonication, lysozyme treatment and/or by freezing and thawing, etc., and a crude extract of polypeptides is then obtained by centrifugation, filtration or the like. The buffer may also contain a protein denaturant such as urea or guanidine hydrochloride, or a surfactant such as Triton X-100™. When the polypeptides of the present invention are secreted in the culture, the bacterial cells are separated from the supernatant by a publicly known method after completion of the culture to collect the supernatant.

The polypeptides contained in the extract or culture supernatant obtained in this manner can be purified by a suitable combination of publicly known methods of isolation and purification. Examples of such publicly known methods of isolation and purification include methods featuring the use of the degree of dissolution such as solvent precipitation or salting out; methods making use of differences primarily in molecular weight such as dialysis, ultrafiltration, gel filtration, and SDS-polyacrylamide gel electrophoresis; methods making use of differences in charge such as ion exchange chromatography; methods making use of specific affinity such as affinity chromatography; methods making use of hydrophobic differences such as reverse phase HPLC; and methods making use of differences in isoelectric point, such as isoelectric point electrophoresis and chromatofocusing.

When the polypeptides of the present invention are obtained in free form, they can be converted to a salt by a publicly known method or a modification thereof. Alternatively, when they are obtained in the form of a salt, they can be converted to free form or another salt by a common method or a modification thereof.

Polypeptides of the present invention produced by recombinants can be modified as desired through the action of suitable protein-modifying enzymes before or after purification, or the polypeptides can be partially removed. Examples of protein-modifying enzymes include trypsin, chymotrypsin, arginylendopeptidase, and protein kinase.

The extracted polypeptides can be activated by refolding the polypeptides. Refolding can be conducted, for example, by publicly known methods described in R.H. Pain, ed., *Folding of Proteins,* 245-279 (1995), Schpringer-Verlag Tokyo, or the modifications thereof. The peptides can be diluted with buffer containing either no, or a low concentration of, extraction agent (such as guanidine hydrochloride, urea or similar caotropic solubilizers, n-lauryl methyl glycine, SDS or similar surfactants, etc.) in one or more steps, and refolding can be brought about by means of dialysis using semipermeable membranes, replacing the buffer through gel filtration, or the like. Arginine, polyethylene glycol, a neutral surfactant, or the like can be added to prevent the polypeptides from aggregating in such cases. Air oxidation, a redox buffer system, or the like can be used for the formation of the polypeptide disulfide bonds. Examples of redox buffers include those based on glutathione, cysteine, dithiothreitol, 2-mercaptoethanol, or cysteamine.

The polypeptides of the present invention are brought into contact with copper ions, calcium ions, or magnesium ions, or substances providing copper ions, substances providing calcium ions, or substances providing magnesium ions (1) after the above isolation and purification, (2) after the refolding process, (3) at the same time as the refolding process, or (4) after the polypeptides have been extracted from the bacterial cells. In such cases, the polypeptides may be brought into contact simultaneously with two or more of the copper ions, calcium ions, or magnesium ions, or substances providing copper ions, substances providing calcium ions, or substances providing magnesium ions.

As used in the present Specification, "purified polypeptides of the present invention" include polypeptides of the present invention which have been purified by the aforementioned methods of isolation and purification (purity of at least 50%, preferably at least 80%, and more preferably at least 90%), as well as crude extracts obtained from recombinant cells, culture supernatant, or partially purified products obtained by the aforementioned isolation or purification.

Copper ions, calcium ions, or magnesium ions, or substances providing copper ions, substances providing calcium ions, or substances providing magnesium ions can be added to the extract or refolding buffer, or to both the extract and refolding buffer, in order to bring the polypeptides into contact with copper, calcium, or magnesium ions, or substances providing copper ions (copper, copper sulfate, copper acetate, copper bromide, copper fluoride, copper oxalate, copper chloride, copper formate, or copper nitrate), substances providing calcium ions (such as calcium, calcium sulfate, calcium acetate, calcium nitrate, calcium formate, or calcium chloride), or substances providing magnesium (such as magnesium, magnesium sulfate, magnesium acetate, magnesium nitrate, magnesium formate, or magnesium chloride) during refolding. The pH of the extract is preferably 4 to 10, more preferably 6 to 9, and even more preferably 7 to 9. The pH of the refolding solution is preferably 3 to 10, more preferably 3 to 8, and even more preferably 4 to 6. In terms of the copper ions, calcium ions, or magnesium ions, or substances providing copper ions, substances providing calcium ions, or substances providing magnesium ions, the concentration is preferably 0.1 to 10000 µM, more preferably 1 to 1000 µM, and even more preferably 1 to 100 µm, when, for example, copper sulfate, calcium chloride, or magnesium sulfate is added to the extract or refolding buffer, or to both the extract and refolding buffer. The extraction may be carried out at 0 to 37°C for 1 to 96 hours, and the refolding may be carried out at 0 to 37°C for 1 to 96 hours.

The polypeptides of the present invention may be brought into contact with copper ions, calcium ions, or magnesium ions, or substances providing copper ions, substances providing calcium ions, or substances providing magnesium ions before or after refolding. Examples of methods for bringing the polypeptides of the present invention into contact with copper, calcium, or magnesium ions, or substances providing copper ions (copper, copper sulfate, copper acetate, copper bromide, copper fluoride, copper oxalate, copper chloride, copper formate, or copper nitrate), substances providing calcium ions (such as calcium, calcium sulfate, calcium acetate, calcium nitrate, calcium formate, or calcium chloride), or substances providing magnesium (such as magnesium, magnesium sulfate, magnesium acetate, magnesium nitrate, magnesium formate, or magnesium chloride) after purification include mixing or dissolving the purified polypeptides of the invention in a solution containing copper ions, calcium ions, or magnesium ions, or substances providing copper ions, substances providing calcium ions, or substances providing magnesium ions (such as pyridine solution, acetic acid buffer, Tris hydroxymethane buffer, and phosphate buffer) at a protein (polypeptide) concentration of 0.01 to 50 mg/mL, preferably 0.1 to 20 mg/mL, and even more preferably 1 to 10 mg/mL for 0.1 to 24 hours of activation at 0 to 37°C. As for the concentration of copper ions, calcium ions, or magnesium ions, or substances providing copper ions, substances providing calcium ions, or substances providing magnesium ions to be added, in the case of copper sulfate, for example, it should be 0.01 to 1000 mM, preferably 0.1 to 100mM, and even more preferably 0.1 to 10 mM.

The purified polypeptides of the present invention can also be brought into contact with copper, calcium, or magnesium ions, or substances providing copper ions (copper, copper sulfate, copper acetate, copper bromide, copper fluoride, copper oxalate, copper chloride, copper formate, or copper nitrate), substances providing calcium ions (such as calcium, calcium sulfate, calcium acetate, calcium nitrate, calcium formate, or calcium chloride), or substances providing magnesium (such as magnesium, magnesium sulfate, magnesium acetate, magnesium nitrate, magnesium formate, or magnesium chloride) during the step for removing the N terminal methionine in accordance with the method in EP 0812856, thereby giving activated polypeptides of the present invention in which the N terminal methionine has been removed.

The polypeptides of the present invention which have been activated in the manner described above have action in suppressing myeloid stem cell colony formation, thus protecting the myeloid stem cells from chemotherapeutic drugs, for example, and can thus be used as a drug or the like to bring about recovery from leukopenia during the administration of anti-tumor agents.

The polypeptides of the present invention can be used as such drugs in the usual manner. For example, they can be orally administered in the form of coated or enterically coated tablets, capsules, elixirs, microcapsules, and the like, and can be parenterally administered in the form of injections such as sterile solutions with water or other pharmaceutically acceptable liquids, or suspensions. Such preparations can be manufactured, for example, by mixing the compounds or salts in generally recognized unit dose formulations along with physiologically acceptable carriers, flavorings, excipients, vehicles, antiseptics, stabilizers, binders, and the like. The content of the active ingredient in such formulations will give a suitable dose within the indicated range.

Examples of additives miscible with tablets, capsules, and the like include binders such as gelatin, corn starch, tragacanth gum, and gum arabic, excipients such as crystalline cellulose, extenders such as corn starch, gelatin, and alginic acid, lubricants such as magnesium stearate, sweeteners such as sucrose, lactose, and saccharin, and flavors such as peppermint, *Akamono* oil, or cherry. In the case of unit formulations in the form of capsule preparations, the aforementioned types of material can also include a liquid carrier such as a lipid. Sterile compositions for injections can be formulated by a common method such as dissolving or suspending a naturally produced vegetable oil or the like such as sesame oil or coconut oil and the active ingredient in a vehicle such as water for injection.

Examples of aqueous solutions for injection include physiological saline, isotonic liquids containing glucose or other adjuvants (such as D-sorbitol, D-mannitol, and sodium chloride). Suitable dissolving aids such as alcohols (such as ethanol), polyalcohols (such as propylene glycol and polyethylene glycol), and nonionic surfactants (such as Polysorbate 80™ and HCO-50) can also be used. Examples of oleaginous solutions include sesame oil and soybean oil. Examples of dissolution aids include benzyl benzoate and benzyl alcohol.

Buffers (such as phosphate buffers and sodium acetate buffers), analgesics (such as benzalkonium chloride and procaine hydrochloride), stabilizers (such as human serum albumin and polyethylene glycol), preservatives (such as benzyl alcohol and phenol), antioxidants, and the like can also be blended. Injections prepared are usually packaged aseptically in suitable ampules.

The resulting preparation is safe and has low toxicity, and can thus be administered, for example, to humans and other mammals (such as mice, rats, guinea pigs, rabbits, goats, pigs, cows, cats, dogs, and monkeys).

The dosage of the polypeptides in the present invention varies depending on the subject's condition, etc. The dosage in the form of an injection, for example, for adult patients with cancer (per 60 kg body weight) is generally about 0.01 to 30 mg at a time, preferably about 0.1 to 20 mg, and even more preferably about 0.1 to 10 mg. The dosage for other mammals can also be calculated in terms of 60 kg.

Abbreviations for bases, amino acids, and the like in the Specification and figures are based on the IUPAC-IUB Commission on Biochemical Nomenclature and on abbreviations common in the field. Examples are given below. Optical isomers of amino acids are the L form, unless otherwise specified.
DNA: deoxyribonucleic acid
cDNA: complementary deoxyribonucleic acid
A: adenine
T: thymine
G: guanine
C: cytosine
RNA: ribonucleic acid
mRNA: messenger ribonucleic acid
dATP: deoxyadenosine triphosphate
dTTP: deoxythymidine triphosphate
dGTP: deoxyguanidine triphosphate
dCTP: deoxycytidine triphosphate
ATP: adenosine triphosphate
EDTA: ethyleendiaminetetraacetic acid
SDS: sodium dodecylsulfate
Gly: gylcine
Ala: alanine
Val: valine
Leu: leucine
Ile: isoleucine
Ser: serine
Thr: threonine
Cys: cysteine
Met: methionine
Glu: glutamic acid
Asp: aspartic acid
Lys: lysine
Arg: arginine
His: histidine
Phe: phenylalanine
Tyr: tyrosine
Trp: tryptophan
Pro: proline
Asn: asparagine
Gln: glutamine

The following are SEQ ID NOsof the Sequence Listing in the present Specification.

### [SEQ ID NO: 1]

The amino acid sequence of MPIF-1Δ23 in the present invention

### [SEQ ID NO: 2]

The base sequence of Primer 1 used in Reference

### Example 1

### [SEQ ID NO: 3]

The base sequence of Primer 2 used in Reference

### Example 1

### [SEQ ID NO: 4]

The base sequence of Primer 3 used in Reference

### Example 1

The *E. coli* (Escherichia coli) transformants MM294(DE3)/pTCIId23-MPIF1 obtained in Reference Example 1 below were deposited with the Deposit No, FERM BP-6582 on November 24, 1998 at the National Institute of Bioscience and Human Technology, Agency of Industrial Science and Technology, Ministry of International Trade and Industry (NIBH), and the Deposit No. IFO 16212 on October 27, 1998 at the Institute for Fermentation, Osaka (IFO).

### Examples

Examples and reference examples are given below to illustrate the present invention in further detail, but the scope of the present invention is not limited by these examples. Genetic manipulation involving the use of *E. coli* was based on the methods described in *Molecular Cloning.*

### Reference Example 1: Construction of MPIF-1Δ23 expressing strain

The structural gene of mature MPIF-1 was amplified by PCR from a human liver cDNA library (Quick-Clone, by Clontech) using a primer 1 (5'-CATATGCGGG TCACAAAAGA TGCAGAGACA GAG: SEQ ID NO: 2) having an NdeI cleavage site and start codon adjacent upstream of the structural gene and a primer 2 (5'-CATATGGGACAGATTCCATGCTACTAGTGCTGA: SEQ ID NO: 3) having a BamHI cleavage site adjacent downstream of a stop codon. The gene thus amplified by PCR was ligated to the pCR2.1 vector using a TA original cloning kit (by Invitrogen) to prepare pCR2.1/MPIF-1. This was introduced to *E. coli* JM109, and transformants were selected using ampicillin resistance and β-galactosidase activity as indicators. Transformants having pCR2.1/MPIF-1 were cultured, and pCR2.1/MPIF-1 was prepared using a QIAprep8 Miniprep kit (by Qiagen).

The structural gene of MPIF-1Δ23 with 23 amino acids deleted from the N terminal of mature MPIF-1 was obtained by PCR from the pCR2.1/MPIF-1. The structural gene of MPIF-1Δ23 was amplified by PCR using primer 2 and a primer 3 (5'-CATATGCGGG TCACAAAAGA TGCAGAGACA GAG: SEQ ID NO: 4) having an NdeI cleavage site and start codon adjacent upstream of the gene encoding the Asp at 24 of mature MPIF-1. The gene thus amplified by PCR was ligated to the pCR2.1 vector using a TA original cloning kit (by Invitrogen) to prepare pCR2.1/d23-MPIF1. This was introduced to *E. coli* JM109, and transformants were selected using ampicillin resistance and β-galactosidase activity as indicators. Transformants having pCR2.1/d23-MPIF1 were cultured, and pCR2.1/d23-MPIF1 was prepared using a QIAprep8 Miniprep kit (by Qiagen).

Expression plasmids for MPIF-1Δ23 were constructed in the following manner. pBR322 was digested with NdeI, the ends were blunted with T4 DNA polymerase (DNA Blunting kit, by Takara Shuzo), and pBRdesNde lacking the NdeI recognition site was prepared by religation. pET3c was digested with Bgl II-EcoRV, approximately 0.26 kbp fragments were recovered, the ends were blunted with T4 DNA polymerase, and pBR/T7 desNde was prepared by ligation with the ScaI fragment of pBRdesNde. pBR322desBam lacking the BamHI recognition site of pBR322 was prepared by site-directed mutagenesis (Quick Change, by Stratagene). The SphI-EcoRV fragment of pBR322desBam was ligated with the SphI-EcoRV fragment of pBR/T7desNde, giving the tetracycline resistance expression vector pTCII. pCR2.1/d23-MPIF1 was digested with NdeI and BamHI for agarose electrophoresis, and the approximately 240 bp MPIF-1Δ23 structural gene was recovered using a QIAquick Spin Purification Kit (by Qiagen). The expression vector pTCII was digested with NdeI and BamHI for agarose electrophoresis, and approximately 4.6 kbp bands were similarly recovered. The MPIF-1Δ23 structural gene was ligated with the NdeI-BamHI fragment of the pTCII expression vector and then incorporated into *E. coli* JM109 for selection of transformants by tetracycline resistance, and plasmids were again recovered from the strain, giving the expression plasmid pTCII/d23-MPIF1.

The pTCII/d23-MPIF1 was introduced into *E. coli* MM294(DE3) for selection of transformants by tetracycline resistance, giving the MPIF-1Δ23 expression line MM294 (DE3)/pTCIId23-MPIF1.

### Reference Example 2: Culture of MPIF-1Δ23

The MPIF-1Δ23 expression strain MM294 (DE3)/pTCIId23-MPIF1 was cultured for 16 hours at 30°C in 1 L of LB medium (1% peptone, 0.5% yeast extract, 0.5% sodium chloride) containing 10 mg/L tetracycline. The resulting culture was used to inoculate 50 L fermentation tanks containing 20 L primary fermenting medium (1.68% sodium monohydrogen phosphate, 0.3% sodium dihydrogen phosphate, 0.1% ammonium chloride, 0.05% sodium chloride, 0.024% magnesium sulfate, 0.02% Nupol LB-625, 0.0005% thiamine hydrochloride, 1.5% glucose, 1.0% casamino acid, 1.0% yeast extract) for aerated stirred culture at 200 rpm, an air flow rate of 16 L/min, and a temperature of 37°C. When the turbidity of the culture reached about 1300 Klett units, 5.95 mg/L portions of isopropyl-β-D-thiogalactopyranoside (IPTG) were added. 0.75% glucose was added 24 minutes, 130 minutes, and 200 minutes after the IPTG was added, and the culture lasted until 9.5 hours after the start of culture. The culture broth was continuously centrifuged at a rate of about 300 mL/min at 12,000 rpm, giving 620 g of cells.

### Reference Example 3: Purification of MPIF-1Δ23

65 g cells obtained in Reference Example 2 was suspended in 65 mL of 0.1 M Tris-HCl (pH 7.5) containing 25 mM EDTA, and the cells were disrupted by continuous ultrasonication using a sonifier (by Bronson). The disrupted cell suspension was centrifuged (20 min at 10,000 rpm), and the supernatant was discarded to obtain inclusions. 130 mL of 0.1 M Tris-HCl (pH 7.5) containing 25 mM EDTA and 3 M guanidine hydrochloride was added to the inclusions to lyse them, and the lysate was centrifuged (20 min at 10,000 rpm). 1.3 L of 0.1 M sodium acetate buffer (pH 4.5) containing 2 mM EDTA and 0.125 M sodium chloride was added to 130 mL of the resulting supernatant for refolding overnight at 4°C, followed by centrifugation (20 min at 10,000 rpm) to give 60 L of supernatant. The resulting supernatant was adsorbed at a flow rate of 20 mL/min onto a heparin Toyopearl column (2.6 cm × 26 cm, by Tosoh) equilibrated with 50 mM phosphate buffer (pH 6.0), which was extensively washed with the buffer used for equilibration and eluted with a 1 M sodium chloride linear gradient at rate of 8 mL/min. Fractions containing the MPIF-1Δ23 were collected and dialyzed over night at 4°C against 50 mM sodium acetate buffer (pH 5.8) using a dialysis membrane for fractions with a molecular weight of 1000, and were added to a TSKgel SP-5PW column (2.15 cm × 15 cm, by Tosoh) equilibrated with 50 mM sodium acetate buffer (pH 5.8). The TSKgel SP-5PW column to which the MPIF-1Δ23 had been adsorbed was eluted with a 1.5 M sodium chloride linear gradient. Fractions containing MPIF-1Δ23 were collected and concentrated by ultrafiltration using Centriprep 3 (fraction molecular weight 3000, by Amicon).

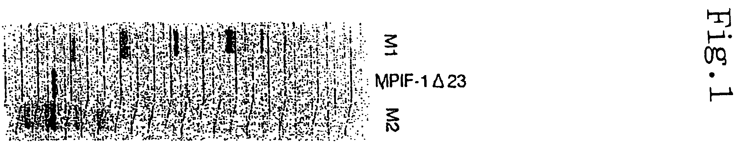

The concentrate was applied to gel filtration using a Sephacryl S100HR column (4.4 cm × 47.3 cm, by Amersham Pharmacia Biotech) equilibrated with 50 mM sodium acetate buffer (pH 5.8) containing 150 mM sodium chloride, resulting in 149 mg of purified MPIF-1Δ23 product. Figure 1 shows the results of SDS-PAGE of the resulting MPIF-1Δ23. The purified MPIF-1Δ23 was hydrolyzed in the vapor phase for 24 and 48 hours at 110°C with 6 N hydrochloric acid containing 4% thioglycolic acid, and the amino acid composition was sequenced using an amino acid sequencer (Hitachi L-8500A Amino Acid Analyzer). As shown in Table 1, the results were consistent with the amino acid composition deduced from the cDNA base sequence.

**Table 1:**

| amino acid composition of MPIF-1Δ23 prepared in Reference Example 3 | | |
|---|---|---|
| Amino acid | MPIF-1Δ23 Theoretical value | Measured value |
| Asx | 7 | 6.8 |
| Thr | 6 | 6.0 |
| Ser | 8 | 7.4 |
| Glx | 5 | 5.0 |
| Pro | 4 | 3.8 |
| Gly | 2 | 2.0 |
| Ala | 3 | 3.0 |
| Val | 3 | 2.8 |
| Met | 3 | 2.9 |
| Ile | 4 | 3.7 |
| Leu | 5 | 5.1 |
| Tyr | 2 | 2.0 |
| Phe | 2 | 2.0 |
| Lys | 7 | 6.7 |
| His | 1 | 1.0 |
| Arg | 7 | 6.9 |
| Ser and Thr extrapolated at 0 hr | | |
| Others are mean at 24 and 48 hr | | |

The N terminal amino acid sequence was sequenced using a vapor phase protein sequencer (Applied Biosystems model 477A). As shown in Table 2, the results were consistent with the amino acid composition deduced from the cDNA base sequence.

**Table 2:**

| N terminal amino acid composition of MPIF-1Δ23 prepared in Reference Example 3 | | |
|---|---|---|
| Residue No. | Detected PTH*amino acids (pmol) | Sequence of MPIF1Δ23 |
| 1 | Met (426) | Met |
| 2 | Asp (379) | Asp |
| 3 | Arg (209) | Arg |
| 4 | Phe (340) | Phe |
| 5 | His (121) | His |
| 6 | Ala (284) | Ala |
| 7 | Thr (117) | Thr |
| 8 | Ser ( 78) | Ser |
| 9 | Ala (144) | Ala |
| 10 | Asp ( 84) | Asp |

| | | |
|---|---|---|
| PTH*: analyzed using 1 nmol phenyl thiohidantoin | | |

### Example 1: Activation of inactive MPIF-1Δ23 by treatment with copper sulfate

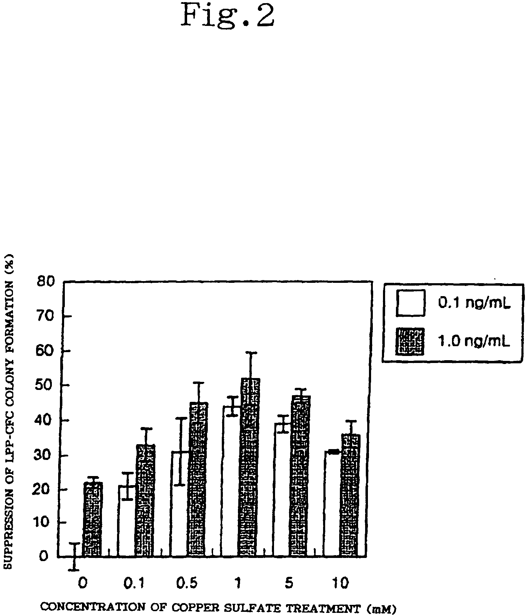

The MPIF-1Δ23 obtained Reference Example 3 was added to a concentration of 0.5 mg/mL in 1.0 M pyridine buffer (pH 5.9) containing 0.1, 0.5, 1, 5, and 10 mM copper sulfate for 1 hour of treatment at room temperature. The buffer was then replaced by means of a NAP-5 column (Amersham Pharmacia Biotech) equilibrated with 50 mM sodium acetate buffer (pH 5.8) containing 150 mM sodium chloride. The action of MPIF-1Δ23 in suppressing mouse myeloid cell differentiation before and after activation was assayed in the following manner. 5 to 12 week old female C57BL/6 mice were asphyxiated using dry ice, and the femur was excised and flushed with 10% FBS-IMDM using a 25 G syringe to extract the myeloid cells from the marrow. The myeloid cell suspension was carefully layered in 15 mL centrifuge tubes containing 4 mL Histopack 1119, and the cells were collected after centrifugation (30 min, 750 g). Cells in the intermediate layer were collected, washed twice with 10% FBS-IMDM, suspended in 10 mL of 10% FBS-IMDM, and used to inoculate Petri dishes (10 cm in diameter) for 2 hours of cell culture at 37°C and 5% CO₂. Petri dishes to which cells other than myeloid cells had adhered were carefully shaken, the culture was transferred to a centrifuge tube, and the cells were collected by centrifugation (5 min at 1000 rpm). Cells were suspended to a concentration of 44,000 cells/mL in 10% FBS-IMDM, and were cultured for 7 days at 37°C, 5% CO₂, and 7% O₂ in complete methyl cellulose Methocult (by Veritas) containing 2.5 ng/mL mouse IL-1α (by Genezyme), 2.5 ng/mL mouse IL-3 (by Upstate Biotechnology), 10 ng/mL mouse SCF (by Genezyme), 10 ng/mL human M-CSF (by Genezyme), and MPIF-1Δ23. Each well was examined by microscope after the culture to count the number of LPP in wells, where a colony diameter under 0.5 mm was regarded as LPP-CFC. The rate at which colony formation was inhabited with the addition of MPIF-1Δ23 is given in percentages, where 100% is the LPP-CFC colony number when no MPIF-1Δ23 was added. The results are shown in Figure 2. LPP-CFC colony formation was inhibited -1% with 0.1 ng/mL MPIF-1Δ23 obtained in Reference Example 3, and 22% with 1.0 ng/mL, indicating virtually no suppression of colony formation, whereas maximum activity was found with 1 mM copper sulfate, resulting in 44% suppression of LPP-CFC colony formation with 0.1 ng/mL and 52% suppression with 1 ng/mL.

### Example 2: Activation of inactive MPIF-1Δ23 by removing N terminal amino acids

25 mg of the MPIF-1Δ23 obtained in Reference Example 3 was added to 25 mL of 1.0 M pyridine buffer (pH 5.9) containing 0.15 M glyoxylic acid and 5 mM copper sulfate for 1 hour of treatment at room temperature, and the buffer was then replaced by means of a Sephadex G-25 column (3.2 x 45 cm, Amersham Pharmacia Biotech) equilibrated with 50 mM sodium acetate buffer (pH 5.8) containing 150 mM sodium chloride. 2 M sodium acetate, 2 M formic acid, and 40 mM phenylene-1,2-diamine were added to 60 mL of the Sephadex G-25 eluate, and a reaction was brought about for 15 hours at 37°C. After completion of the reaction, the reaction solution was concentrated to about 50 mL using a YM-3 ultrafiltration membrane (by Amicon), and the buffer was replaced by means of a Sephadex G-25 column (3.2 × 45 cm, Amersham Pharmacia Biotech) equilibrated with 50 mM sodium acetate buffer (pH 5.8) containing 150 mM sodium chloride. 60 mL of the Sephadex G-25 eluate was adsorbed to a TSKgel CM-5PW column (2.15 × 15 cm, by Tosoh) equilibrated with 50 mM sodium acetate buffer (pH 5.8) containing 150 mM sodium chloride, and was eluted with a 1.5 M sodium chloride linear gradient. Fractions containing MPIF-1Δ23 were collected and concentrated by ultrafiltration to a concentration of about 3 mL using a Centriprep 3 (fraction molecular weight 3000, by Amicon). The concentrate was applied to gel filtration on a Hi-Load 16/60 Superdex 75 column (2.6 cm × 60 cm, Amersham Pharmacia Biotech) equilibrated with 50 mM sodium acetate buffer (pH 5.8) containing 150 mM sodium chloride, giving 8.5 mg of purified MPIF-1Δ23 (Des-Met MPIF-1Δ23) lacking the N terminal methionine. The resulting purified Des-Met MPIF-1Δ23 was hydrolyzed in the vapor phase for 24 and 48 hours at 110°C with 6 N hydrochloric acid containing 4% thioglycolic acid, and the amino acid composition was sequenced using an amino acid sequencer (Hitachi L-8500A Amino Acid Sequencer). As shown in Table 3, the results were consistent with the amino acid composition deduced from the cDNA base sequence.

**Table 3:**

| amino acid composition of Des-Met MPIF-1Δ23 prepared in Example 2 | | |
|---|---|---|
| Amino acid | MPIF-1Δ23 Theoretical value | Measured value |
| Asx | 7 | 7.2 |
| Thr | 6 | 5.4 |
| Ser | 8 | 8.3 |
| Glx | 5 | 5.0 |
| Pro | 4 | 3.8 |
| Gly | 2 | 2.1 |
| Ala | 3 | 2.9 |
| Val | 3 | 3.0 |
| Met | 3 | 2.0 |
| Ile | 4 | 3.7 |
| Leu | 5 | 5.2 |
| Tyr | 2 | 2.0 |
| Phe | 4 | 3.9 |
| Lys | 7 | 6.9 |
| His | 1 | 1.1 |
| Arg | 7 | 6.8 |
| Ser and Thr extrapolated at 0 hr | | |
| Others are mean at 24 and 48 hr | | |

The N terminal amino acid sequence was then sequenced using a vapor phase protein sequencer (Applied Biosystems model 477A). As shown in Table 4, the results were consistent with the amino acid composition deduced from the cDNA base sequence.

**Table 4:**

| N terminal amino acid composition of Des-Met MPIF-1Δ23 prepared in Example 2 | | |
|---|---|---|
| Residue No. | Detected PTH* amino acids (pmol) | Sequence of MPIF1△23 |
| 1 | Asp (272) | Met |
| 2 | Arg (238) | Asp |
| 3 | Phe (390) | Arg |
| 4 | His ( 81) | Phe |
| 5 | Ala (193) | His |
| 6 | Thr ( 71) | Ala |
| 7 | Ser ( 49) | Thr |
| 8 | Ala ( 55) | Ser |
| 9 | Asp ( 51) | Ala |
| 10 | - | Asp |

| | | |
|---|---|---|
| PTH*: analyzed using 1 nmol phenyl thiohidantoin | | |

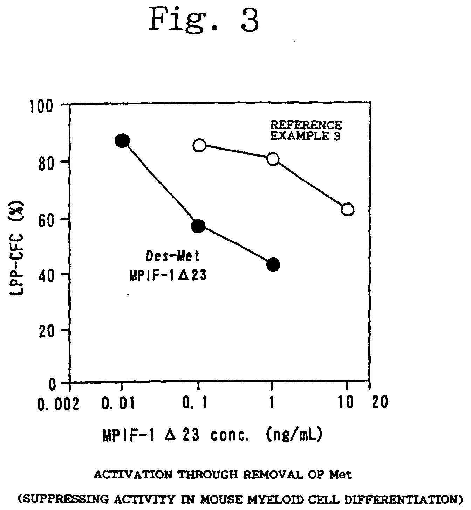

Both analyses confirmed that the N terminal methionine had been removed. Figure 3 gives the results obtained in the assay of the suppression of mouse myeloid cell differentiation. The Des-Met MPIF-1Δ23 prepared by the above method showed higher activity than the unactivated MPIF-1Δ23 obtained in Reference Example 3.

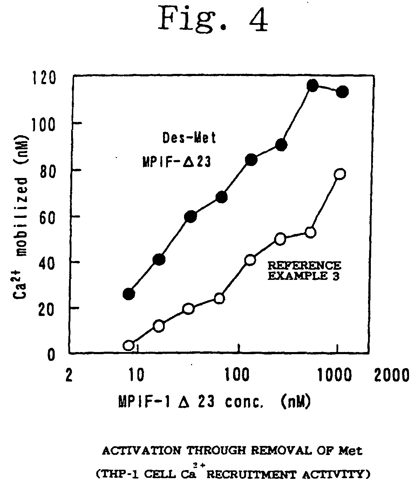

The intracellular recruitment of calcium ions was assayed in the following manner. THP-1 cells (ATCC, TIB-202) were suspended to a concentration of 5 × 10⁶ cells/mL in MG buffer (modified Gey's Buffer) containing 1 mM calcium chloride, the fluorescence indicator Fura PE3/AM (Wako Pure Chemicals) was added to a concentration of 2 µM, and the suspension was maintained for 30 minutes at 37°C. The cells were washed with MB buffer, then suspended to a concentration of 2.5 × 10⁶ cells/mL in MG buffer containing 1 mM calcium chloride, and aliquoted in 100 µL portions into 96-well plates. The MPIF-1Δ23 obtained in Reference Example 3 and the Des-Met MPIF-1Δ23 obtained above were added to the well, and the changes in the intracellular calcium ion concentration were measured using a fluorescence drug screening system (Hamamatsu Photonics). The results are given in Figure 4. The Des-Met MPIF-1Δ23 obtained above had higher activity than the unactivated MPIF-1Δ23 obtained in Reference Example 3.

### Example 3

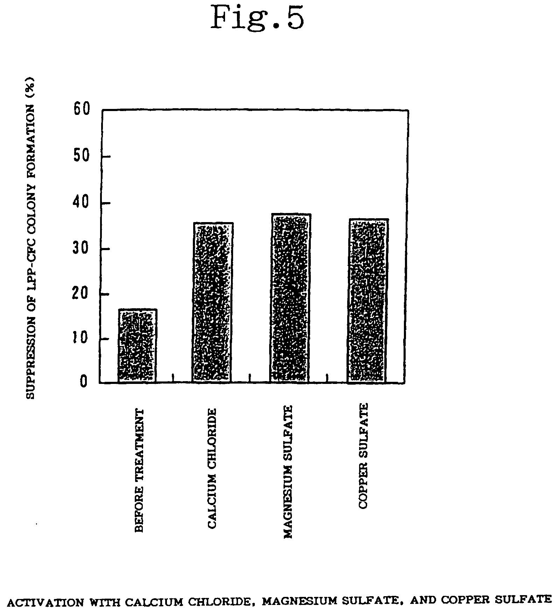

The MPIF-1Δ23 obtained in Reference Example 3 was added to a concentration of 0.5 mg/mL in 50 mM sodium acetate buffer (pH 5.8) containing 1 mM copper sulfate, calcium chloride, or magnesium sulfate for 1 hour of treatment at room temperature, and the buffer was then replaced by means of a NAP-5 column (Amersham Pharmacia Biotech) equilibrated with 50 mM sodium acetate buffer (pH 5.8) containing 150 mM sodium chloride. The activity of MPIF-1Δ23 in suppressing mouse myeloid cell differentiation before and after activation was assayed in accordance with the method described in Example 1. All levels of activity were higher than that of the unactivated MPIF-1Δ23 obtained in Reference Example 3 (Figure 5).

### INDUSTRIAL APPLICABILITY

The methods for activation or manufacturing of the present invention enable the inexpensive mass production of active MPIF-1Δ23 which protects myeloid stem cells from chemotherapeutic drugs and permits rapid recovery from leukopenia, which is a limiting factor in the administration of anti-tumor agents.

## Claims

1. A process for activating a purified polypeptide, **characterized by** bringing a purified polypeptide having an amino acid sequence identical or substantially identical to the amino acid sequence represented by SEQ ID NO: 1, into contact with copper ions, calcium ions, or magnesium ions, or a substance providing copper ions, a substance providing calcium ions, or a substance providing magnesium ions.

2. The process according to Claim 1, wherein the substance providing copper ions is copper, copper sulfate, copper bromide, copper fluoride, copper oxalate, copper chloride, copper formate, copper acetate, or copper sulfide.

3. The process according to Claim 2, wherein the substance providing copper ions is copper sulfate.

4. The process according to Claim 1, wherein the substance providing calcium ions is calcium, calcium sulfate, calcium acetate, calcium nitrate, calcium formate, or calcium chloride.

5. The process according to Claim 4, wherein the substance providing calcium ions is calcium chloride.

6. The process according to Claim 1, wherein the substance providing magnesium ions is magnesium, magnesium sulfate, magnesium acetate, magnesium nitrate, magnesium formate, or magnesium chloride.

7. The process according to Claim 6, wherein the substance providing the magnesium ion is magnesium sulfate.

8. A process according to Claim 1, wherein the activity of the activated polypeptide is activity in suppressing cell differentiation.

9. A process for producing an active polypeptide, **characterized by** bringing a purified polypeptide having an amino acid sequence identical or substantially identical to the amino acid sequence represented by SEQ ID NO: 1 into contact with copper ions, calcium ions, or magnesium ions, or a substance providing copper ions, a substance providing calcium ions, or a substance providing magnesium ions.

10. A polypeptide, or an amide thereof, or an ester thereof, or a salt thereof, which has been activated by means of the activating process according to Claim 1.
